# EUROPÄISCHE PATENTSCHRIFT

(11) **EP 3 909 060 B1**
(45) Veröffentlichungstag und Bekanntmachung des Hinweises auf die Patenterteilung: **09.09.2026**
(21) Anmeldenummer: 20701532.2
(22) Anmeldetag: 07.01.2020
(51) Int. Cl.: G16H 50/20, G16H 40/63, G16H 50/30, G16H 50/70, A61B 5/00, H04R 25/00, A61B 5/01, A61B 5/021, A61B 5/024, A61B 5/0531, A61B 5/08, A61B 5/145, A61B 5/282, A61B 5/291, A61B 5/1455

(54) **VERFAHREN ZUR BEREITSTELLUNG VON DATEN FÜR EINE SCHNITTSTELLE**
METHOD OF PROVIDING DATA FOR AN INTERFACE
MÉTHODE DE FOURNITURE DE DONNÉES POUR UNE INTERFACE

(30) Priorität: 07.01.2019 DE 102019200100
(43) Veröffentlichungstag der Anmeldung: 17.11.2021
(73) Patentinhaber: Cosinuss GmbH, 81379 München (DE)
(72) Erfinder: KREUZER, Johannes, 81377 München (DE)
(74) Vertreter: Franke, Dirk
(86) Internationale Anmeldenummer: PCT/EP2020/050227
(87) Internationale Veröffentlichungsnummer: WO 2020/144189

(56) Entgegenhaltungen:
- US-A1- 2013 343 585
- US-A1- 2018 263 562
- US-A1- 2018 288 533

## Beschreibung

### Technisches Gebiet

Die Erfindung betrifft ein Verfahren zur Bereitstellung von Daten für eine Schnittstelle, wobei die Schnittstelle für eine Computer-implementierte Gesundheitsüberwachung mittels mindestens einer in ein Hörgerät integrierten elektronischen Vorrichtung eingerichtet ist. Die Erfindung betrifft ferner ein Datenverarbeitungssystem, ein Computerprogramm und ein Computer-lesbares Speichermedium.

### Stand der Technik

Verfahren zur Bereitstellung von Daten für eine Schnittstelle sind aus dem Stand der Technik bekannt. Insbesondere sind derartige Verfahren und die entsprechenden Schnittstellen in Systemen für eine Computer-implementierte Gesundheitsüberwachung bekannt. Ebenfalls aus dem Stand der Technik sind Hörgeräte, mittels derer über mindestens eine in ein Hörgerät integrierte elektronische Vorrichtung einzelne Gesundheitsparameter bzw. gesundheitliche Aspekte eines Trägers des Hörgeräts überwacht werden können.

Nachteilig sind einige der Überwachungssysteme technisch aufwendig realisiert und umfassen eine Vielzahl einzelner Komponenten. Die EP 3035710 A1 beschreibt ein System zur Überwachung des Status und/oder der Leistung eines oder mehrerer Hörgeräte, wobei das System mehrere Zugangspunkte zum Empfangen drahtlos übertragener Signale umfasst, die wiederum mit einer Zentraleinheit kommunikativ verbunden sind, die Zentraleinheit ist schließlich mit dem Internet/Cloud verbunden. Die US 2013/0343585 A1 beschreibt ein Verfahren für eine Hörhilfe-Vorrichtung mit einem medizinischen Sensor, wobei in mehreren Kommunikations-Schritten aufgrund von erfassten Sensordaten Funktionen in der Hörhilfe-Vorrichtung ausgeführt werden können. Die US 2018/288533 A1 beschreibt ein Überwachungssystem, das so konfiguriert ist, dass es den Status und/ oder die Funktion eines oder mehrerer Hörgeräte in einer Umgebung überwacht. Die US 2018/263562 A1 beschreibt ein Hörsystem, bestehend aus einem linken und einem rechten Hörgerät, die an der linken und rechten Seite des Kopfes eines Benutzers getragen werden, und mit Sensoren zur Überwachung gesundheitsbezogener Parameter des Benutzers.

### Darstellung der Erfindung, Aufgabe, Lösung, Vorteile

Ausgehend von den vorgenannten Verfahren und Vorrichtungen des Standes der Technik liegt der vorliegenden Erfindung die Aufgabe zugrunde, ein Verfahren und mit dem Verfahren assoziierte Systeme und Programme einen bereitzustellen, welche/s die Nachteile nicht aufweist/en. Insbesondere soll ein Verfahren eine Datenbereitstellung mit nur wenigen Verfahrensschritten ermöglichen und die assoziierten Systeme sollen technisch einfach zu realisieren sein.

Das erfindungsgemäße Verfahren soll die einfache Interaktion eines Verwenders des erfindungsgemäßen Verfahrens mit einer Schnittstelle ermöglichen, so dass unterschiedliche Konfigurationen einer in einem Hörgerät angeordneten Sensoranordnung in einem Schritt ausgewählt und die in einem Zeitintervall erfassten, gesundheitsbezogenen Daten auf einfache Art und Weise an die Schnittstelle übertragen werden können.

Die der Erfindung zugrunde liegende Aufgabe wird durch das in den Ansprüchen definierte Verfahren und die assoziierten Systeme und Programme gelöst, wie sich auch aus dem beiliegenden Ausführungsbeispiel ergibt.

In einem ersten Aspekt betrifft die Erfindung daher ein Verfahren zur Bereitstellung von Daten für eine Schnittstelle, wobei die Schnittstelle für eine Computer-implementierte Gesundheitsüberwachung mittels mindestens einer in ein Hörgerät integrierten, elektronischen Vorrichtung eingerichtet ist, umfassend mehrere Schritte, wobei in einem ersten Schritt eine Anordnung von einem oder mehreren, in einem Hörgerät angeordneten Sensoren S₁-Sᵢ zur Erfassung gesundheitsbezogener Daten zum Auswählen durch einen Verwender der gesundheitsbezogenen Daten bereitgestellt wird. Hörgeräte zur Versorgung der häufigsten Form von Schwerhörigkeit, der sensorineuralen Schwerhörigkeit, sind aus dem Stand der Technik bekannt. Bauformen dieser Hörgeräte sind Hinterdem-Ohr- und Im-Ohr-Geräte, wobei letztere in einer Vielzahl unterschiedlicher Formen mit unterschiedlichem Grad der baulichen Miniaturisierung vorliegen. Unter dem Begriff "gesundheitsbezogene Daten" werden erfindungsgemäß physiologische Daten eines Trägers des Hörgeräts verstanden, welche sich mittels externer Sensoren erfassen lassen, beispielsweise mit der Herzfrequenz oder dem Blutdruck assoziierte Daten. Erfindungsgemäß sind ein oder mehrere Sensoren S₁-Sᵢ im Hörgerät angeordnet. Das erfindungsgemäße Verfahren kann jedoch auch von zusätzlichen Sensoren erfasste Daten berücksichtigen, wobei die zusätzlichen Sensoren Daten an anderen, externen Messpunkten des Trägers des Hörgeräts erfassen, beispielsweise am Handgelenk über ein entsprechendes Armband oder am Brustkorb über einen entsprechenden Brustgurt. Das erfindungsgemäße Verfahren kann auch Daten berücksichtigen, welche von zusätzlichen, intern lokalisierten Sensoren erfasst werden. In einem zweiten Schritt des erfindungsgemäßen Verfahrens wird eine Anordnungs-Auswahl eines Verwenders erfasst, wobei es sich beim Verwender und beim Träger des Hörgerätes um unterschiedliche Personen handeln kann. Beispielsweise wird aus einer Sensoranordnung der Sensoren S₁-Sᵢ eine Auswahl der Sensoren S₁, S₂ und S₄ durch den Verwender erfasst. Die ausgewählten Sensoren können dabei mit einem Gesundheits-Parameter assoziierte, physiologische Daten auf unterschiedliche Art und Weise erfassen, z.B. den Gesundheit-Parameter "Herzfrequenz" über unterschiedliche Modalitäten der entsprechenden Sensoren. Die ausgewählten Sensoren können auch physiologische Daten unterschiedlicher Modalität erfassen, z.B. Herzfrequenz und Körpertemperatur. In einem weiteren Schritt werden die gesundheitsbezogenen Daten eines Trägers des Hörgeräts mittels des einen oder der mehreren, vom Verwender ausgewählten Sensoren in einem Zeitintervall erfasst. Das Zeitintervall kann dabei voreingestellt sein, z.B. in Abhängigkeit von der Art der gesundheitsbezogenen Daten (z.B. Erfassung mehrerer EKG Intervalle), oder von einem Verwender des erfindungsgemäßen Verfahrens entsprechend ausgewählt werden. Im letzten Verfahrensschritt erfolgt die Übertragung der erfassten Daten an eine Schnittstelle. Unter einer Schnittstelle wird dabei vorzugsweise eine Softwareschnittstelle verstanden, wobei die Softwareschnittstelle ein logischer Berührungspunkt in einem Softwaresystem ist, über welche der Austausch von Kommandos und Daten zwischen verschiedenen Prozessen und Komponenten ermöglicht und geregelt wird. Solche Schnittstellen sind aus dem Stand der Technik bekannt und werden grundsätzlich differenziert in datenorientierte Schnittstellen, welche nur zur Kommunikation benutzt werden, und funktionsorientierte Schnittstellen zur Ausführung bestimmter Funktionalitäten, welche primär beteiligte Systemteile synchronisieren oder unterstützen.

Das erfindungsgemäße Verfahren ermöglicht die einfache Konfiguration der in einem Hörgerät eines Trägers angeordneten Sensoren zur Erfassung gesundheitsbezogener Daten durch einen Verwender. Beispielsweise können gesundheitsbezogene Daten eines Trägers an einem ersten Ort erfasst und an einen Verwender an einem zweiten Ort zur Analyse übermittelt werden. Das erfindungsgemäße Verfahren ermöglicht ferner die simultane Konfiguration und Erfassung gesundheitsbezogener Daten durch mehrere Verwender, gegebenenfalls an unterschiedlichen Orten.

In einer bevorzugten Weiterbildung des erfindungsgemäßen Verfahrens können die Sensordaten mit einer Tastfrequenz (sampling rate) von bis zu 1 kHz erfasst werden. Die Übertragung kann mit einer Übertragungsfrequenz von bis zu 1 kHz erfolgen. Die Tastfrequenz von bis zu 1 kHz ermöglicht eine Erfassung gesundheitsbezogener Daten, welche eine erhöhte Aussagekraft hinsichtlich davon abgeleitete physiologischer Parameter erlaubt, beispielsweise des Blutdrucks aus der mittels eines Drucksensors erfassten Druckkurve. Die Übertragung der Daten an die Schnittstelle kann dabei kontinuierlich oder in Paketen erfolgen.

In einer weiteren Implementierung des erfindungsgemäßen Verfahrens können die gesundheitsbezogenen Daten aus der Gruppe umfassend Elektrokardiogramm und/oder Herzfrequenz und/oder Pulsfrequenz und/oder Blutdruck und/oder Körpertemperatur und/oder Hautleitfähigkeit und/oder Blutzuckerwert und/oder elektrische Hirnaktivität und/oder Sauerstoffsättigung und/oder Atemfrequenz ausgewählt sein. Die jeweiligen gesundheitsbezogenen, physiologischen Daten sind aus dem Stand der Technik bekannt. Mittels des erfindungsgemäßen Verfahrens können auf einfache Art und Weise simultan oder nacheinander unterschiedliche gesundheitsbezogene Daten des Trägers des Hörgeräts an eine Schnittstelle übertragen werden, welche dann durch einen oder mehrere Verwender des erfindungsgemäßen Verfahrens abrufbar sind.

In einer bevorzugten Ausführungsform können die Sensoren S₁-Sᵢ ausgewählt sein aus der Gruppe umfassend PPG-Sensor, EKG/EEG-Sensor, Drucksensor, Temperatursensor, Leitfähigkeitssensor, Feuchtigkeitssensor, Glukosesensor. Ein herkömmlicher PPG-Sensor (Photoplethysmographie, PPG) liegt dabei direkt auf der Haut und besteht aus einer Lichtquelle, welche Gewebe und Blutgefäße durchdringendes Licht einer bestimmten Anwendungswellenlänge aussendet, und einem Photodetektor. Ein herkömmlicher EKG (Elektrokardiogramm)- Sensor kann mit mindestens zwei kleinen Elektroden zum Erfassen elektrischer Herzsignale ausgebildet sein; ein herkömmlicher EEG (Elektroencephalographie)-Sensor kann mit mindestens zwei kleinen Elektroden zum Erfassen von Hirnströmen ausgebildet sein; ein Temperatursensor erfasst die Körpertemperatur durch die Infrarotstrahlung des Gewebes oder durch ein Kontakttemperaturfühler. Alle vorgenannten Sensoren sind aus dem Stand der Technik bekannt und so miniaturisiert bzw. miniaturisierbar, dass sie in einem herkömmlichen Hörgerät der oben genannten Bauweise angeordnet werden und bei geringstmöglicher Interferenz gesundheitsbezogene Daten eines Trägers des Hörgeräts erfassen können. Insbesondere bevorzugt können die Sensoren S₁-Sᵢ in einem hinter dem Ohr zu tragenden Hörgerät angeordnet sein, wodurch die Interaktion zwischen den separaten Sensorsignalen minimiert wird.

In einer vorteilhaften Weiterbildung des erfindungsgemäßen Verfahrens können in einem weiteren Schritt die erfassten Daten in einem in das Hörgerät integrierten Speicher erfasst werden. Das Speichermedium ist aus dem Stand der Technik bekannt und kann eine oder mehrere Arten von Speichern umfassen, einschließlich RAM oder Flash usw., wobei der Speicher flüchtig oder nicht flüchtig sein kann. Mittels eines derartigen Speichers können auch in einem oder mehreren vergangenen Zeitintervallen erfasste Daten an die Schnittstelle übermittelt und vom Verwender abgerufen werden.

In einer weiteren Ausführungsform kann die Anordnungs-Auswahl über die Schnittstelle erfolgen und die Anordnungs-Auswahl die Schnittstelle über drahtlose Kommunikation einer mit dem Hörgerät verbundenen, externen Rechnervorrichtung erreichen. Die drahtlose Kommunikation kann dabei mittels herkömmlicher Kommunikationstechnologie erreicht werden, beispielsweise über Bluetooth oder ANT+ oder WiFi oder NFC oder über eine Funkzugangstechnologie (radio access technology, RAT) wie z.B. NOMA (nonorthogonal multiple access), oder über eine entsprechend ausgebaute, RATbasierte Kommunikationsarchitektur mit unterschiedlichen Stationen, welche eine geringe Signalinterferenz mit nahtloser Übertragung und hoher Kapazität verbindet.

In einer bevorzugten Weiterbildung kann das Verfahren gemäß Angaben des Verwenders automatisierbar sein. Das Verfahren kann somit zu festgelegten Zeitpunkten ausgeführt werden, so dass Daten vom Verwender kalkulierbar für die Schnittstelle übertragen werden, beispielsweise einmal / Stunde, oder einmal / Tag.

In einer weiteren Implementierung des erfindungsgemäßen Verfahrens kann das Verfahren bei einer Änderung eines Aktivitätszustands des Trägers automatisch ausgeführt werden, wobei der Aktivitätszustand des Trägers über einen Beschleunigungssensor kontinuierlich erfasst wird. Als mikro-elektromechanisches System (MEMS) ausgebildete Beschleunigungssensoren sind im Bereich der medizinischen Überwachung aus dem Stand der Technik bekannt. MEMS zeichnen sich durch hohe Zuverlässigkeit aus und, infolge ihrer geringen Größe, durch hohe Messgeschwindigkeit.

In einer weiteren Implementierung des erfindungsgemäßen Verfahrens kann das Verfahren nach dem Schritt des Erfassens von gesundheitsbezogenen Daten eines Trägers des Hörgeräts mittels des einen oder der mehreren, vom Verwender ausgewählten Sensoren in einem Zeitintervall zusätzliche Schritte umfassen, nämlich zunächst das Bestimmen, aus den Sensordaten zweier oder mehrerer Sensoren S₁, S₂-Sᵢ, eines Datenmusters D₁, D₂-Dᵢ des Trägers desHörgeräts, wobei jeder Sensor S₁, S₂-Sᵢ mit einem entsprechenden Sensorindex SI₁, SI₂-SIᵢ assoziiert ist. Mittels Berechnungsmitteln kann mindestens ein Gesundheitsindikator des Trägers des Hörgeräts berechnet werden und zwar als Funktion des mindestens einen Datenmusters und einem multidimensionalen Muster gesundheitsbezogener Daten, wobei eine erste Dimension Zeit und mindestens eine zweite Dimension der Sensorindex SI₁, SI₂-SIᵢ ist, wobei der Gesundheitsindikator und/oder das Datenmuster und/oder die erfassten Daten an die Schnittstelle übertragen werden. Erfindungsgemäß wird unter einem Gesundheitsindikator ein Hinweis auf einen bestimmten Zustand eines Gesundheitsparameters des Trägers des Hörgeräts verstanden. Beispielsweise kann ein auf den Blutdruck des Trägers bezogener Gesundheitsindikator indizieren, dass der Blutdruck im Normbereich liegt, oder aber dass er, hinsichtlich des gemessenen Zeitintervalls, zu hoch (Hypertonie) oder zu niedrig (Hypotonie) ist. In einem weiteren Schritt kann mittels Überprüfungsmitteln überprüft werden, ob der berechnete Gesundheitsindikator plausibel ist, nämlich dann, wenn Sensordaten von Sensoren S₁, S₂-Sᵢ mit unterschiedlicher Modalität verwendet werden. Beispielsweise kann eine Herzfrequenz des Trägers des Hörgeräts mittels der Daten eines PPG Sensors oder eines EKG Sensors oder eines Drucksensors ermittelt werden. Sollten aus den entsprechenden Daten unterschiedliche, nichtplausible Werte für den Gesundheitsindikator Herzfrequenz ermittelt werden, kann dem Träger des Hörgeräts und/oder dem Verwender des Verfahrens die fehlende Plausibilität über entsprechende Kommunikationsmittel signalisiert werden. Insbesondere bevorzugt kann der mindestens eine Gesundheitsindikator einen Messwert der Qualität der Messwerte sowie der Gesundheit des Trägers im Zeitintervall angeben.

In einer besonders bevorzugten Implementierung des erfindungsgemäßen Verfahrens kann das multidimensionale Muster gesundheitsbezogener Daten ein Träger- spezifisches Muster sein, welches durch eine Abfolge von Verfahrensschritten generiert werden kann. Zunächst erfolgt das Erfassen von Sensordaten eines Trägers eines Hörgeräts in einem ersten Zeitintervall, wobei die Sensordaten Daten von einem oder mehreren, in einem Hörgerät eines Trägers angeordneten Sensoren S₁-Sᵢ zur Erfassung gesundheitsbezogener Daten sind. Aus diesen Sensordaten des einen oder der mehreren Sensoren S₁-Sᵢ kann in einem nachfolgenden Schritt ein jeweiliges Datenmuster D₁-Dᵢ des Trägers im ersten Zeitintervall bestimmt werden, gefolgt von dem Schritt des Erfassens von Sensordaten des Trägers eines Hörgeräts in mindestens einem weiteren Zeitintervall. Aus den Sensordaten des einen oder der mehreren Sensoren S₁-Sᵢ kann ein weiteres, jeweiliges Datenmusters D₁-Dᵢ des Trägers in dem mindestens einen, weiteren Zeitintervall bestimmt werden. Mittels Berechnungsmitteln kann dann ein Durchschnitts-Datenmusters DD₁-DDᵢ des Verwenders aus den in den vorhergehenden Bestimmungs-Schritten bestimmten Datenmustern berechnet werden, um das Träger-spezifische, multidimensionale Muster gesundheitsbezogener Daten des Trägers zu erhalten. Das vorgenannte Verfahren zur Generierung des multidimensionalen Datenmusters ermöglicht eine optimale Anpassung des Gesundheitsindikators an den Träger. Dies ist beispielsweise dann vorteilhaft, wenn das Träger-spezifische multidimensionale Datenmuster von einem aus allgemein verfügbaren Gesundheitsdaten abgeleiteten, multidimensionalen Datenmuster in einer Weise abweicht, die zwar signifikant, aber für den Träger nicht pathologisch und damit ohne Krankheitsrelevanz ist. In einer alternativen Ausführungsform kann das multidimensionale Muster gesundheitsbezogener Daten eine Verweistabelle allgemeiner, physiologischer Daten sein. Eine derartige Tabelle bezieht gesundheitsbezogene Daten einer Vielzahl von Personen ein und enthält ich Norm bzw. Durchschnittswerte, welche beispielsweise hinsichtlich Alter und Geschlecht sortiert sind.

In einer insbesondere bevorzugten Implementierung des erfindungsgemäßen Verfahrens können Berechnungsmittel im Hörgerät, in einer mobilen Rechnereinheit und/oder in einer Zentraleinheit angeordnet sein, besonders bevorzugt können Berechnungsmittel im Hörgerät oder in einer mobilen Rechnereinheit angeordnet sein, am meisten bevorzugt können Berechnungsmittel in einer mobilen Rechnereinheit angeordnet sein. Wenn im Hörgerät Berechnungsmittel angeordnet sind, kann ein Teil der Datenverarbeitung bereits vorteilhaft im Hörgerät erfolgen, insbesondere hängt die Anordnung der Berechnungsmittel aber von der Anzahl und Dimensionierung der Sensoren ab, da zusätzlich zu den Sensoren, den Kommunikations- und datenverarbeitenden Strukturen entsprechend energieliefernde Vorrichtungen vorzusehen sind. Vorzugsweise kann für die zusätzlichen Sensoren und Übertragung die Energieversorgung des Hörgeräts verwendet werden; oder aber die Energie wird durch eine lokale Energiegewinnungsvorrichtung zur Energieversorgung von Sensoren und Strukturen bereitgestellt, beispielsweise in der Form eines miniaturisierten, thermoelektrischen Generators. In einer vorteilhaften Weiterbildung des erfindungsgemäßen Verfahrens kann die Schnittstelle in einer mobilen Rechnereinheit und/oder in einer Zentraleinheit angeordnet sein.

In einer weiteren Ausführungsform des erfindungsgemäßen Verfahrens kann das Verfahren einen Authentifizierungsschritt umfassen. Unter einer Authentifizierung wird dabei eine Verifizierung der Behauptung der Authentizität verstanden, mittels derer der Verwender des Verfahrens angibt, ob er über eine Zugangsberechtigung zu den Daten verfügt. Beispielsweise kann der Verwender zunächst einen Benutzernamen angeben und sich zusätzlich authentisieren, indem er ein Passwort angibt. Vorteilhaft kann die Authentifizierung zu einem frühen Verfahrens-Zeitpunkt erfolgen, nämlich bereits nach dem Erfassen und vor dem Übertragen der gesundheitsbezogenen Daten an die Schnittstelle. Alternativ kann die Authentifizierung auch nach dem Übertragen der erfassten Daten erfolgen. Ein weiterer Schutz der gesundheitsbezogenen Daten des Trägers des Hörgeräts kann durch eine Verschlüsselung der Daten erreicht werden; hier können die Daten vorteilhaft vor dem Übertragen der Daten an die Schnittstelle verschlüsselt werden.

In einem zweiten Aspekt betrifft die Erfindung ein Datenverarbeitungssystem, welches Mittel zum Ausführen des vorgehend beschriebenen, erfindungsgemäßen Verfahrens umfasst. Unter einem Datenverarbeitungssystem wird dabei ein Computer, eine Rechenanlage oder ein Rechensystem verstanden. Definitionsgemäß ist ein Datenverarbeitungssystem eine Funktionseinheit zur Verarbeitung von Daten, wobei als Verarbeitung die Durchführung mathematischer, umformender, übertragender oder speichernder Operationen definiert ist. Das Datenverarbeitungssystem gemäß der vorliegenden Erfindung kann beispielsweise unterschiedliche Auswahlmittel, Erfassungsmittel, Übertragungsmittel und/oder Berechnungsmittel umfassen.

In einem dritten Aspekt betrifft die Erfindung ein Computerprogramm, welches Instruktionen umfasst, die bei Ausführung des Programms auf einem Computer, den Computer dazu veranlassen, das vorstehend beschriebene Verfahren auszuführen.

In einem vierten Aspekt betrifft die Erfindung einen Computer-lesbares Speichermedium, welches Instruktionen umfasst, die bei Ausführung des Programms auf einem Computer, den Computer dazu veranlassen, dass vorstehend beschriebene Verfahren auszuführen.

Es wird ein bevorzugtes Ausführungsbeispiel beschrieben, auf welches die Erfindung jedoch nicht beschränkt ist. Grundsätzlich kann jede im Rahmen der vorliegenden Anmeldung beschriebene bzw. angedeutete Variante der Erfindung besonders vorteilhaft sein, je nach wirtschaftlichen, technischen und gegebenenfalls medizinischen Bedingungen im Einzelfall. Soweit nichts Gegenteiliges dargelegt ist, bzw. soweit grundsätzlich technisch realisierbar, sind einzelne Merkmale der beschriebenen Ausführungsformen austauschbar oder miteinander sowie mit per se aus dem Stand der Technik bekannten Merkmalen kombinierbar.

### Kurze Beschreibung der Figur

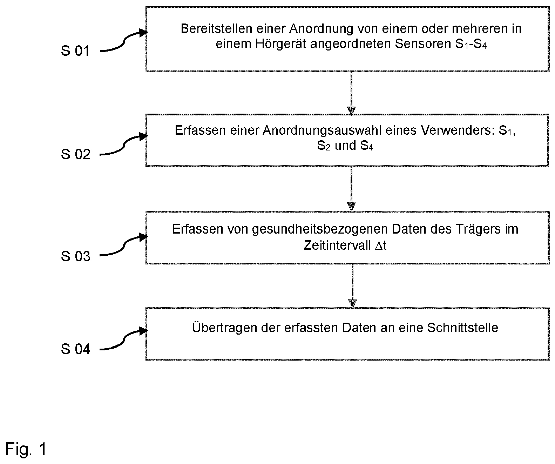

Figur 1 zeigt ein Flussdiagramm des erfindungsgemäßen Verfahrens.

### Detaillierte Beschreibung

Die Erfindung betrifft ein Verfahren zur Bereitstellung von Daten für eine Schnittstelle, wobei die Schnittstelle für eine Computer-implementierte Gesundheitsüberwachung mittels mindestens einer in ein Hörgerät integrierten elektronischen Vorrichtung eingerichtet ist. Das Verfahren umfasst mehrere Schritte, wobei in einem ersten Schritt (S 01) eine Anordnung von einem oder mehreren, in einem Hörgerät angeordneten, hier z- B. Sensoren S₁-S₄ zur Erfassung gesundheitsbezogener Daten zum Auswählen durch einen Verwender der gesundheitsbezogenen Daten bereitgestellt wird. Die S₁-S₄ können beispielsweise als ein PPG-Sensor, ein Drucksensor, ein Temperatursensor und ein Leitfähigkeitssensor ausgebildet sein. Dementsprechend können vorliegend gesundheitsbezogene Daten hinsichtlich Sauerstoffsättigung, Blutdruck, Körpertemperatur und Hautleitfähigkeit erfasst werden. Alle vorgenannten Sensoren sind aus dem Stand der Technik bereits in miniaturisierter Form bekannt so dass sie in einem herkömmlichen Hörgerät, z.B. in einem hinter dem Ohr zu tragenden Hörgerät zur Versorgung der sensorineuralen Schwerhörigkeit, angeordnet werden und bei geringstmöglicher Interferenz gesundheitsbezogene Daten eines Trägers des Hörgeräts erfassen können. In einem zweiten Schritt (S 02) wird eine Anordnungs-Auswahl eines Verwenders erfasst, beispielsweise werden aus einer Senderanordnung der Sensoren S₁-S₄ die Sensoren S₁, S₂ und S₄ ausgewählt. In einem dritten Schritt (S03) werden die gesundheitsbezogenen Daten eines Trägers des Hörgeräts mittels des einen oder der mehreren, vom Verwender ausgewählten Sensoren in einem Zeitintervall Δt erfasst. Das Zeitintervall kann dabei voreingestellt sein, beispielsweise in Abhängigkeit der Art der gesundheitsbezogenen Daten, oder von einem Verwender des erfindungsgemäßen Verfahrens entsprechend ausgewählt werden. Im vierten Schritt (S04) erfolgt die Übertragung der erfassten Daten an eine Schnittstelle. Unter einer Schnittstelle wird dabei vorzugsweise eine Softwareschnittstelle verstanden, wobei die Softwareschnittstelle einen logischen Berührungspunkt in einem Softwaresystem ist, welche den Austausch von Kommandos und Daten zwischen verschiedenen Prozessen und Komponenten ermöglicht und regelt.

## Patentansprüche

1. Verfahren zur Bereitstellung von Daten für eine Schnittstelle, wobei die Schnittstelle für eine Computer-implementierte Gesundheitsüberwachung mittels mindestens einer in ein Hörgerät integrierten elektronischen Vorrichtung eingerichtet ist, umfassend:
01. Bereitstellen einer Anordnung von einem oder mehreren, in einem Hörgerät angeordneten Sensoren S₁-Sᵢ zur Erfassung gesundheitsbezogener Daten zum Auswählen durch einen Verwender der gesundheitsbezogenen Daten,
02. Erfassen einer Anordnungs-Auswahl eines Verwenders,
03. Erfassen von gesundheitsbezogenen Daten eines Trägers des Hörgeräts mittels des einen oder der mehreren, vom Verwender ausgewählten Sensoren in einem Zeitintervall,
Übertragen der erfassten Daten an eine Schnittstelle;
wobei das Verfahren die zusätzlichen Schritte umfasst:
03.' Bestimmen, aus den erfassten Sensordaten zweier oder mehrerer Sensoren S₁, S₂-Sᵢ, eines Datenmusters D₁, D₂-Dᵢ des Trägers des Hörgeräts, wobei jeder Sensor S₁, S₂-Sᵢ mit einem entsprechenden Sensorindex SI₁, SI₂-SIᵢ assoziiert ist,
03." Berechnen, mittels Berechnungsmitteln, mindestens eines
Gesundheitsindikators des Trägers als Funktion des mindestens einen Datenmusters und einem multidimensionalen Muster
gesundheitsbezogener Daten, wobei eine erste Dimension Zeit und
mindestens eine zweite Dimension der Sensorindex SI₁, SI₂-SIᵢ ist,
wobei der Gesundheitsindikator und/oder das Datenmuster und/oder
die erfassten Daten an die Schnittstelle übertragen werden, und
03. ‴ Überprüfung, mittels Überprüfungsmitteln, einer Plausibilität des Gesundheitsindikators unter Verwendung der Sensordaten von Sensoren S₁, S₂-Sᵢ mit unterschiedlicher Modalität.

2. Verfahren gemäß Anspruch 1, wobei die Sensordaten mit einer Tastfrequenz von bis zu 1 kHz erfasst werden und wobei die Übertragung mit einer Übertragungsfrequenz von bis zu 1 kHz erfolgt.

3. Verfahren gemäß einem der Ansprüche 1 oder 2, wobei die gesundheitsbezogenen Daten ausgewählt sind aus der Gruppe umfassend Elektrokardiogramm, und/oder Herzfrequenz und/oder Pulsfrequenz und/oder Blutdruck und/oder Körpertemperatur und/oder Hautleitfähigkeit und/oder Blutzuckerwert und/oder elektrische Hirnaktivität und/oder Sauerstoffsättigung und/oder Atemfrequenz.

4. Verfahren gemäß einem der vorhergehenden Ansprüche, wobei die Sensoren S₁-Sᵢ ausgewählt sind aus der Gruppe umfassend PPG-Sensor, EKG/EEG-Sensor, Drucksensor, Temperatursensor, Leitfähigkeitssensor, Feuchtigkeitssensor, Glukosesensor.

5. Verfahren gemäß einem der vorhergehenden Ansprüche, zusätzlich umfassend den Schritt
02.' Speichern der erfassten Daten in einem in das Hörgerät integrierten Speicher.

6. Verfahren gemäß Anspruch 1, wobei die Anordnungs-Auswahl über die Schnittstelle erfolgt und wobei die Anordnungs-Auswahl die Schnittstelle über drahtlose Kommunikation einer mit dem Hörgerät verbundenen, externen Rechnervorrichtung erreicht.

7. Verfahren gemäß einem der vorhergehenden Ansprüche, wobei das Verfahren gemäß Angaben des Verwenders automatisierbar ist.

8. Verfahren gemäß einem der vorhergehenden Ansprüche, wobei das Verfahren bei einer Änderung eines Aktivitätszustands des Trägers automatisch ausgeführt wird, wobei der Aktivitätszustand des Trägers über einen Beschleunigungssensor kontinuierlich erfasst wird.

9. Verfahren gemäß Anspruch 1, wobei das multidimensionale Muster gesundheitsbezogener Daten ein Träger- spezifisches Muster ist, welches bereitgestellt wird durch die Schritte
1. Erfassen von Sensordaten eines Trägers eines Hörgeräts in einem ersten Zeitintervall, wobei die Sensordaten Daten von einem oder mehreren, in einem Hörgerät eines Trägers angeordneten Sensoren S₁-Sᵢ zur Erfassung gesundheitsbezogener Daten sind,
2. Bestimmen, aus den Sensordaten des einen oder der mehreren Sensoren S₁-Sᵢ, eines jeweiligen Datenmusters D₁-Dᵢ des Trägers im ersten Zeitintervall,
3. Erfassen von Sensordaten des Trägers eines Hörgeräts in mindestens einem weiteren Zeitintervall,
4. Bestimmen, aus den Sensordaten des einen oder der mehreren Sensoren S₁-Sᵢ, eines jeweiligen Datenmusters D₁-Dᵢ des Trägers in dem mindestens einen, weiteren Zeitintervall,
5. Berechnen, mittels Berechnungsmitteln, eines Durchschnitts-Datenmusters DD₁-DDᵢ des Verwenders aus den in Schritt (b) und Schritt (d) bestimmten Datenmustern zum Erhalten des multidimensionalen Musters gesundheitsbezogener Daten des Trägers.

10. Verfahren gemäß einem der Ansprüche 1 oder 9, wobei das multidimensionale Muster gesundheitsbezogener Daten eine Verweistabelle allgemeiner, physiologischer Daten ist.

11. Verfahren gemäß einem der Ansprüche 1, 9 oder 10,
wobei der mindestens eine
Gesundheitsindikator einen Messwert der Qualität der Messwerte sowie der Gesundheit des Trägers im Zeitintervall angibt.

12. Datenverarbeitungssystem umfassend Mittel zum Ausführen des Verfahrens gemäß einem der Ansprüche 1 bis 11.

13. Computerprogramm umfassend Instruktionen, welche, bei Ausführung des Programms auf einem Computer, den Computer dazu veranlassen, das Verfahren gemäß einem der Ansprüche 1 bis 11 auszuführen.

14. Computer-lesbares Speichermedium umfassend Instruktionen, welche, bei Ausführung des Programms auf einem Computer, den Computer dazu veranlassen, das Verfahren gemäß einem der Ansprüche 1 bis 11 auszuführen.

## Claims

1. A method for providing data for an interface, wherein the interface is configured for computer-implemented health monitoring by means of at least one electronic apparatus integrated in a hearing aid, comprising:
01. providing an arrangement of one or more sensors S₁-Sᵢ, which are arranged in a hearing aid and are designed to record health-related data, for selection by a user of the health-related data;
02. recording a user's choice of arrangement;
03. recording health-related data of a wearer of the hearing aid by means of the one or more sensors, selected by the user, in a time interval;
transmitting the recorded data to an interface;
wherein the method comprises the additional steps:
03.' determining, from the recorded sensor data of two or more sensors S₁, S₂-Sᵢ, a data pattern D₁, D₂-Dᵢ of the wearer of the hearing aid, wherein each sensor S₁, S₂-Sᵢ is associated with a corresponding sensor index SI₁, SI₂-SIᵢ;
03.'' calculating, by means of calculation means, at least one health indicator of the wearer as a function of the at least one data pattern and a multidimensional pattern of health-related data, wherein a first dimension is time and at least a second dimension is the sensor index SI₁, SI₂-SIᵢ, wherein the health indicator and/or the data pattern and/or the recorded data are transmitted to the interface; and
03.‴ checking, by means of checking means, a plausibility of the health indicator using the sensor data from sensors S₁, S₂-Sᵢ with different modalities.

2. The method according to claim 1, wherein the sensor data are recorded at a sampling rate of up to 1 kHz, and wherein the transmission takes place at a transmission rate of up to 1 kHz.

3. The method according to claim 1 or 2, wherein the health-related data are selected from the group comprising electrocardiogram, and/or heart rate and/or pulse rate and/or blood pressure and/or body temperature and/or skin conductivity and/or blood sugar level and/or electrical brain activity and/or oxygen saturation and/or respiratory rate.

4. The method according to any one of the preceding claims, wherein the sensors S₁-Sᵢ are selected from the group comprising PPG sensor, ECG/EEG sensor, pressure sensor, temperature sensor, conductivity sensor, moisture sensor, glucose sensor.

5. The method according to any one of the preceding claims, additionally comprising the step
02.' storing the recorded data in a memory integrated in the hearing aid.

6. The method according to claim 1, wherein the choice of arrangement is made via the interface, and wherein the choice of arrangement reaches the interface via wireless communication from an external computer device connected to the hearing aid.

7. The method according to any one of the preceding claims, wherein the method can be automated according to the user's specifications.

8. The method according to any one of the preceding claims, wherein the method is executed automatically when an activity state of the wearer changes, wherein the activity state of the wearer is continuously recorded via an acceleration sensor.

9. The method according to claim 1, wherein the multidimensional pattern of health-related data is a wearer-specific pattern, which is provided by the steps
1. recording sensor data of a wearer of a hearing aid in a first time interval, wherein the sensor data are data from one or more sensors S₁-Sᵢ, arranged in a hearing aid of a wearer, for recording health-related data;
2. determining, from the sensor data of the one or more sensors S₁-Sᵢ, a respective data pattern D₁-Dᵢ of the wearer in the first time interval;
3. recording sensor data of the wearer of a hearing aid in at least one further time interval;
4. determining, from the sensor data of the one or more sensors S₁-Sᵢ, a respective data pattern D₁-Dᵢ of the wearer in the at least one further time interval;
5. calculating, by means of calculation means, an average data pattern DD₁-DDᵢ of the user from the data patterns determined in step (b) and step (d) in order to obtain the multidimensional pattern of health-related data of the wearer.

10. The method according to claim 1 or 9, wherein the multidimensional pattern of health-related data is a reference table of general, physiological data.

11. The method according to claim 1, 9 or 10, wherein the at least one health indicator specifies a measured value of the quality of the measured values and also of the health of the wearer in the time interval.

12. A data processing system comprising means for executing the method according to any one of claims 1 to 11.

13. A computer program comprising instructions which, when the program is run on a computer, cause the computer to execute the method according to any one of claims 1 to 11.

14. A computer-readable storage medium comprising instructions which, when the program is run on a computer, cause the computer to execute the method according to any one of claims 1 to 11.

## Revendications

1. Procédé de fourniture de données pour une interface, l'interface étant configurée pour une surveillance de santé mise en œuvre par ordinateur au moyen d'au moins un dispositif électronique intégré dans une aide auditive, comprenant :
01. la fourniture d'un agencement d'un ou de plusieurs capteurs S₁-Sᵢ agencés dans une aide auditive pour l'acquisition de données relatives à la santé, en vue d'une sélection par un utilisateur des données relatives à la santé,
02. l'acquisition d'une sélection d'agencement d'un utilisateur,
03. l'acquisition de données relatives à la santé d'un porteur de l'aide auditive au moyen du ou des capteurs sélectionnés par l'utilisateur, dans un intervalle de temps,
la transmission des données acquises à une interface ;
le procédé comprenant les étapes supplémentaires suivantes :
03.' la détermination, à partir des données de capteur acquises de deux capteurs ou plus S₁, S₂-Sᵢ, d'un motif de données D₁, D₂-Dᵢ du porteur de l'aide auditive, chaque capteur S₁, S₂-Sᵢ étant associé à un indice de capteur correspondant SI₁, SI₂-SIᵢ,
03.'' le calcul, au moyen de moyens de calcul, d'au moins un indicateur de santé du porteur en fonction de l'au moins un motif de données et d'un motif multidimensionnel de données relatives à la santé, une première dimension étant le temps et au moins une deuxième dimension étant l'indice de capteur SI₁, SI₂-SIᵢ, l'indicateur de santé et/ou le motif de données et/ou les données acquises étant transmis à l'interface, et
03.‴ la vérification, au moyen de moyens de vérification, d'une plausibilité de l'indicateur de santé en utilisant les données de capteur de capteurs S₁, S₂-Sᵢ de modalité différente.

2. Procédé selon la revendication 1, dans lequel les données de capteur sont acquises à une fréquence d'échantillonnage allant jusqu'à 1 kHz et dans lequel la transmission s'effectue à une fréquence de transmission allant jusqu'à 1 kHz.

3. Procédé selon la revendication 1 ou 2, dans lequel les données relatives à la santé sont choisies dans le groupe comprenant l'électrocardiogramme, et/ou la fréquence cardiaque et/ou la fréquence du pouls et/ou la pression artérielle et/ou la température corporelle et/ou la conductivité cutanée et/ou le taux de glycémie et/ou l'activité cérébrale électrique et/ou la saturation en oxygène et/ou la fréquence respiratoire.

4. Procédé selon l'une quelconque des revendications précédentes, dans lequel les capteurs S₁-Sᵢ sont choisis dans le groupe comprenant un capteur PPG, un capteur ECG/EEG, un capteur de pression, un capteur de température, un capteur de conductivité, un capteur d'humidité, un capteur de glucose.

5. Procédé selon l'une quelconque des revendications précédentes, comprenant en outre l'étape
02.' le stockage des données acquises dans une mémoire intégrée dans l'aide auditive.

6. Procédé selon la revendication 1, dans lequel la sélection d'agencement s'effectue via l'interface et dans lequel la sélection d'agencement parvient à l'interface par communication sans fil d'un dispositif informatique externe relié à l'aide auditive.

7. Procédé selon l'une quelconque des revendications précédentes, dans lequel le procédé peut être automatisé selon les indications de l'utilisateur.

8. Procédé selon l'une quelconque des revendications précédentes, dans lequel le procédé est exécuté automatiquement lors d'un changement d'un état d'activité du porteur, l'état d'activité du porteur étant acquis en continu au moyen d'un capteur d'accélération.

9. Procédé selon la revendication 1, dans lequel le motif multidimensionnel de données relatives à la santé est un motif spécifique au porteur, qui est fourni par les étapes
1. l'acquisition de données de capteur d'un porteur d'une aide auditive dans un premier intervalle de temps, les données de capteur étant des données d'un ou de plusieurs capteurs S₁-Sᵢ agencés dans une aide auditive d'un porteur pour l'acquisition de données relatives à la santé,
2. la détermination, à partir des données de capteur du ou des capteurs S₁-Sᵢ, d'un motif de données respectif D₁-Dᵢ du porteur dans le premier intervalle de temps,
3. l'acquisition de données de capteur du porteur d'une aide auditive dans au moins un autre intervalle de temps,
4. la détermination, à partir des données de capteur du ou des capteurs S₁-Sᵢ, d'un motif de données respectif D₁-Dᵢ du porteur dans l'au moins un autre intervalle de temps,
5. le calcul, au moyen de moyens de calcul, d'un motif de données moyen DD₁-DDᵢ de l'utilisateur à partir des motifs de données déterminés à l'étape (b) et à l'étape (d) pour obtenir le motif multidimensionnel de données relatives à la santé du porteur.

10. Procédé selon la revendication 1 ou 9, dans lequel le motif multidimensionnel de données relatives à la santé est une table de référence de données physiologiques générales.

11. Procédé selon la revendication 1, 9 ou 10, dans lequel l'au moins un indicateur de santé indique une valeur de mesure de la qualité des valeurs de mesure ainsi que de la santé du porteur dans l'intervalle de temps.

12. Système de traitement de données comprenant des moyens pour exécuter le procédé selon l'une quelconque des revendications 1 à 11.

13. Programme d'ordinateur comprenant des instructions qui, lorsque le programme est exécuté sur un ordinateur, amènent l'ordinateur à exécuter le procédé selon l'une quelconque des revendications 1 à 11.

14. Support de stockage lisible par ordinateur comprenant des instructions qui, lorsque le programme est exécuté sur un ordinateur, amènent l'ordinateur à exécuter le procédé selon l'une quelconque des revendications 1 à 11.
